# EUROPEAN PATENT APPLICATION

(11) **EP 4 759 228 A1**
(43) Date of publication of application: **17.06.2026**
(21) Application number: 25223614.6
(22) Date of filing: 15.12.2025
(51) Int. Cl.: A61B 5/026, A61B 5/00, A61M 1/36, A61M 60/515, A61M 60/562

(54) **METHOD AND SYSTEM FOR REAL-TIME IDENTIFICATION OF CEREBRAL AUTOREGULATION IMPAIRMENT DURING CARDIAC SURGERY WITH CARDIOPULMONARY BYPASS**

(30) Priority: 13.12.2024 US 202463733915 P
(71) Applicant: Kaunas University of Technology, 44249 Kaunas (LT)
(72) Inventor: Ragauskas, Arminas, LT-51362 Kaunas (LT); Petkus, Vytautas, LT-48300 Kaunas (LT); Chaleckas, Edvinas, LT-50374 Kaunas (LT)
(74) Representative: Sayettat, Julien Christian

(57) **Abstract**

A system and method using rectangular blood flow modulation in a heart-lung machine to non-invasively identify cerebral autoregulation impairment in real-time during coronary artery bypass grafting surgery.

## Description

### FIELD OF THE INVENTION

The present technology related to real time measurement of cerebral autoregulation impairment during surgery. More particularly, the present technology relates to a system and method using rectangular blood flow modulation in a heart-lung machine to identify cerebral autoregulation impairment during coronary artery bypass grafting surgery.

This application claims the benefit of U.S. Provisional Application No. 63/733,915, filed December 13, 2024 the entirety of which is incorporated herewith.

### BACKGROUND OF THE INVENTION

The use of cardiopulmonary bypass (CPB) while treating heart diseases by coronary artery bypass grafting (CABG) surgery has been associated with postoperative cognitive dysfunction (POCD) and postoperative delirium, affecting up to 70% and 53% of patients, respectively. Impaired cerebral autoregulation (CA) during CPB is recognized as one of significant contributing factors to those complications.

Coronary artery bypass grafting (CABG) is one of the most performed major elective surgeries worldwide, accounting for up to 30 % of all cardiac surgeries. CABG surgery involves using a heart-lung machine that provides CPB, ensuring oxygenation and continuous oxygen blood supply to the brain and other vital organs. Up to 70% of CPB patients experience POCD, including memory impairment, concentration difficulties, and a decline in intellectual performance. Additionally, up to 53 % of patients experience postoperative delirium, characterized by acute confusion and associated with extended hospital stays complications and an increased risk of mortality. If not treated early, delirium can lead to further cognitive decline. Although results vary between studies, there is a consensus on the urgent need for solutions to reduce these complications.

Numerous studies have been conducted worldwide to identify factors influencing the outcomes of patients undergoing cardiac surgery with CPB. CA, the mechanism responsible for maintaining stable cerebral blood flow (CBF) despite fluctuations in systemic blood flow and pressure, is recognized as one of the key factors influencing patient outcome following cardiac surgery with CPB. Impaired CA during CPB is found to be associated with a risk of cerebral ischemic or hyperemic injury leading to cognitive impairment and delirium. Therefore, real-time monitoring CA status is recommended as a measure enabling the management of adequate brain perfusion for brain protection during surgery and for improvement of the patient's outcome. Presently, various neuroprotection strategies during CPB have been studied but still there is no consensus on optimal personalized and precise brain protection against ischemic and hyperemic injuries.

Continuous CA status assessment in patients with traumatic brain injuries is used to optimize cerebral perfusion following the idea that the brain is optimally perfused when the CA assessment index PRx is minimal. PRx index shows a phase shift between arterial blood pressure slow waves and invasively recorded intracranial pressure (ICP) slow waves. Invasive monitoring of PRx is an excessive intervention during cardiac surgery. Noninvasive CA assessment indexes like Mx, Cox, VRx use ICP surrogates, such as cerebral blood flow velocity or slow fluctuations in intracranial blood volume. However, continuous and reliable individual patient-specific CA assessment during CPB surgeries is only possible with blood flow waves of the needed shape are generated by the heart-lung machine. What is needed is a method and system for real-time monitoring of CA during CPB using blood flow modulation in a heart-lung machine (HLM) to identify CA impairment events with sub-minute temporal resolution, to immediately restore a patient's CA by timely managing the patient's arterial blood pressure (ABP) thus reducing incidences of POCD and delirium.

### SUMMARY OF THE INVENTION

The invention includes a novel mode of HLM operation for continuous real-time CA monitoring. Presently available heart-lung machines can operate in non-pulsatile and pulsatile blood flow modes. Several clinical studies have employed an experimental slow sinusoidal one-frequency wave-modulated blood flow mode of HLM operation, which was used for continuous CA assessment. One such sinusoidal wave-modulated blood flow mode creates a sinusoidal arterial blood pressure ("ABP") wave in the patient's body, enabling continuous CA status monitoring. However, when using that mode delays of 5 minutes or more were observed between the onset of ischemic or hyperemic events and the triggering of CA impairment start alarms in these studies due to the time needed for brain physiological monitoring data accumulation. This creates a risk of neuronal damage. To prevent neuronal damage, the delay time must be substantially reduced to a matter of seconds, enabling the identification of individual patient-specific lower and upper limits of CA in real-time and to enable immediate restoration of CA through rapid ABP adjustment to stop the ischemic or hyperemic brain injuries according to precise personalized medicine concept.

Seeking to implement fast and reliable, almost real-time CA status monitoring during CPB, the HLM management was modified by introducing a novel periodic rectangular blood flow modulation mode. Such rectangular modulation with a 15 to 30-second period enables real-time monitoring of CA system's output transient responses and detection of the beginning and ending moments of CA impairment with sub-minute temporal resolution. CA status in the current clinical trial was identified according to the shape of CA transient responses. Transient responses are the intracranial blood flow autoregulatory reactions following each front of the rectangular ABP(t) pulses as a series of CA system's input step functions.

### BRIEF DESCRIPTION OF THE DRAWINGS

**FIG. 1** are graphs showing the transient response of the normalized CBFV(t) signal as a reaction to a rectangular pulse in the ABP (t) signal after the rising and falling fronts in the case of intact CA.
**FIG. 2** are graphs showing the transient response of the normalized CBFV(t) signal as a reaction to rectangular pulses in the ABP (t) signal after the rising and falling fronts in the case of intact impaired CA.
**FIG. 3** is an embodiment of a modulation device connected to a heart lung machine for continuous modulation of blood flow with rectangular pulses.
**FIG. 4** are graphs showing the duration of longest CA impairment events in seconds for various patient groups.
**FIG. 5** are graphs showing the lower limits of cerebral autoregulation for three different patients.
**FIG. 6** is an algorithm of the method for using mean arterial blood pressure management in order to restore patient specific cerebral perfusion pressure.

### DETAILED DESCRIPTION OF THE INVENTION

In the field of measuring impaired CA, transient CA system response analysis is widely used. However, a non-invasive CA transient response monitor providing rapid feedback to the heart-lung machine's blood flow controller to manage the mean arterial blood pressure value over time ABP(t) is a novel method to shorten ischemic and hyperemic episodes up to sub-minute durations. This personalized method of optimizing individual patient-specific brain perfusion can help reduce the rate of postoperative cognitive dysfunction and delirium.

A clinical trial was conducted on 108 patients, who underwent elective CABG on-pump surgery. 78 patients were included in the final analysis. A rectangular blood flow modulation technique enabled real-time detection of CA impairments.

The trial showed that a single CA impairment event lasting longer than 241 seconds was statistically significantly associated with POCD (p=0.0178), while impairments exceeding 262 seconds were related to delirium (p=0.0315). The trial demonstrated that CA impairment events and patient-specific lower and upper limits of CA can be identified with sub-minute delays during cardiac surgery with CPB. Lower and upper limits of CA mean critical ABP values CA can be identified by comparing of transient responses of CA to a rising and falling fronts of rectangular ABP(t) pulses which follow rectangular modulation of blood flow of heart and lung machine. ABP(t) critical value at which CA transient response changes from exponential (intact CA) to rectangular (impaired CA) is a marker of intact CA limit.

The study demonstrates the operability of a novel heart and lung machine operation mode with rectangular blood flow modulation. Precise personal ABP(t) mean value management can be performed during CPB to restore patient-specific optimal brain perfusion with sub-minute time resolution.

The trial involved 108 patients undergoing standard elective CABG surgery using CPB who meet inclusion criteria: carotid artery atherosclerosis ≤50%, age 55-90 years. The exclusion criteria included the use of agents affecting the central nervous system (CNS), preoperative neurological or mental disorders and uncontrolled diabetes.

Demographic and clinical data of the patients in the study are shown in Tables 1 and 2. Table 1 shows demographic and clinical data of cardiac surgery patients. Patients are grouped into those with no delirium and those with post-operative delirium.

**TABLE 1**

| Parameter | No Delirium | Delirium | Chi-square critical value | p |
|---|---|---|---|---|
| Numbers | 70 | 8 | | |
| F/M | 13/57 | 2/6 | | 0.646 |
| Age, year | 67, (8) | 64.5, (9) | - | 0.478 |
| Mean, SD, IQR | [62-74] | [57-70] | | |
| MAP, mmHg | 61.1, (4.1) | 66.6, (3.6) | 65.7 | 0.0017 |
| Mean, SD, IQR | [58.1-63.5] | [63.9-68.6] | | |
| LCAI, s | 218, (148) | 376, (271) | 262 | 0.0315 |
| Mean, SD, IQR | [122-241] | [176-465] | | |

MAP - mean arterial blood pressure averaged per group, SD - standard deviation is presented in (), IQR - the interquartile range is presented in [ ], LCAI - the duration of longest cerebral autoregulation impairment event, p - the probability of the Mann-Whitney U or Fisher-exact tests for detecting differences between the groups.

Table 2 is a table with the demographic and clinical data of cardiac surgery patients. Patients are grouped into those who experienced POCD and/or delirium and those without signs of cognitive deterioration.

**TABLE 2**

| Parameter | No Deterioration | POCD and/or Delirium | Chi-square critical value | p |
|---|---|---|---|---|
| Numbers | 51 | 27 | | |
| F/M | 11/40 | 4/23 | - | 0.558 |
| Age, year | 69, (8) | 63, (8) | 64.9 | 0.002 |
| | [64-75] | [55-67.5] | | |
| MAP, mmHg | 60.9, (4.2) | 63.0, (4.3) | - | 0.108 |
| Mean, SD, IQR | [57.9-63.7] | [60.6 65.8] | | |
| LCAI, s | 213, (132) | 327, (240) | 241 | 0.018 |
| Mean, SD, IQR | [121-263] | [155-465] | | |

MAP - mean arterial blood pressure averaged per group, SD - standard deviation is presented in (), IQR - the interquartile range is presented in [ ], LCAI - the duration of longest cerebral autoregulation impairment event, p - the probability of the Mann-Whitney U or Fisher-exact tests for detecting differences between the groups.

All patients underwent routine, surgical, and anesthetic techniques during the CABG surgery with CPB. Premedication included 1-2.5 mg of lorazepam on the eve of the surgery and half the usual dose of metoprolol next morning. Anesthesia was induced with fentanyl (1-2 µg/kg), propofol (2 mg/kg), and rocuronium (0.6 mg/kg) after preoxygenation with 80% oxygen. Maintenance during anesthesia included sevoflurane (BIS 40-60), fentanyl (10-12 µg/kg), and muscle relaxants as needed. Ventilation with 50% oxygen maintained normocapnia (ETCO2 35-45 mmHg). A middle sternotomy and standard anastomosis techniques were performed. Systemic heparinization (3mg/kg) was given for and ACT ≥400s. The CPB circuit was primed with 1500 ml Ringer's acetate and 1000 IU heparin, maintaining a cardiac index of 2.2 - 2.4. L/min/m2 at 35-36 o C. Cold St. Thomas cardioplegia (1000 ml initially, 500 ml every 35 - 40 min) provided myocardial protection. Protamine sulfate (1.2 ml/kg) reversed anticoagulation, with active clotting time controlled at 120-140 s. Tranexamic acid (1g) was administered thrice per clinical protocol. Standard monitoring of vital signs included an electrocardiogram, heart rate, invasive arterial blood pressure, pulse oximetry, esophageal and body core temperature, central venous pressure and urine output.

All patients underwent a neurocognitive evaluation with a standardized neuropsychological test before and 7 - 10 days after surgery. The same qualified examiner performed all evaluations. The assessment included measures of the Addenbrooke test (ACE - III) and the Hopkins Verbal Learning Test - Revised (HVLT-R). The Confusion Assessment Method for the Intensive Care Unit (CAM - ICU) was used to identify postoperative delirium. Postoperative cognitive dysfunction (POCD in Table 2) including 8 patients with delirium (POCD and/or Delerium in Table 2) was found in 27 patients out of the 78 patients analyzed as shown in Table 2.

All patients underwent a neurocognitive evaluation with a standardized neuropsychological test before and 7 - 10 days after surgery. The same qualified examiner performed all evaluations. The assessment included measures of the Addenbrooke test (ACE - III) and the Hopkins Verbal Learning Test - Revised (HVLT-R). The Confusion Assessment Method for the Intensive Care Unit (CAM - ICU) was used to identify postoperative delirium. Postoperative cognitive dysfunction (POCD in Table 2) including 8 patients with delirium was found in 27 patients out of the 78 patients analyzed.

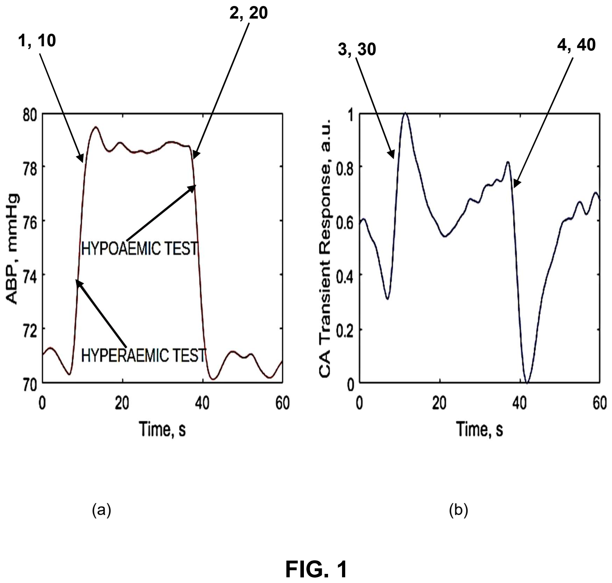

**FIG. 1** shows monitoring results of the novel mode of HLM operation-blood flow with rectangular modulation-that was applied for real-time CA status monitoring during the CPB. A heart lung machine (Stöckert S5 HLM (LivaNova), was used for the CPB procedure, which included a novel mechatronic plug-in device, or module **30** for continuous modulation of blood flow with rectangular pulses having a 30-second duration. The system includes an arterial blood pressure monitor for obtaining ABP(t) signals from the patient. The system can also include an intracranial blood flow monitor velocity monitor for obtaining CA transient responses following each discreet from of the ABP(t) signals downstream from the patient. Rectangular pulses having as low as a 15 second duration could also be used because a transient CA response in a healthy brain (cardiac bypass surgery patients have the healthy brains before surgery) has a duration of less than 15 seconds.

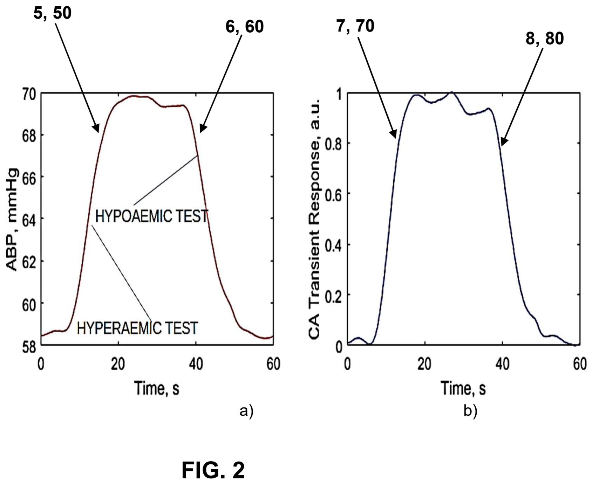

**FIGS. 1** and **2** show the transient responses of the normalized cerebral blood flow velocity as a function of time CBFV(t) signal as reactions on rectangular pulses in the arterial blood pressure (ABP) signal over time (ABP(t) after the rising and falling fronts in the cases of intact and impaired CA. The rising fronts **1, 3, 5** and **7,** can be seen in **FIGS. 1a, 1b****,** **2a** and **2b** respectively. The falling fronts **2, 4, 6** and **8** can be seen in **FIGS 1a, 1b****,** **2a** and **2b** respectively. The durations of transient responses are at most 15 seconds. This results in the reduction of time delay between the start moments of cerebral ischemic or hyperemic events and the start of an alarm, which requires immediate reaction in ABP management to restore intact CA and to stop brain ischemia or hyperemia. The rectangular modulation with a 15 to 30-second period enables real-time monitoring of CA system's output transient responses and real-time detection of the beginning and ending moments of CA impairment with sub-minute temporal resolution. With the rectangular pulses having a 30-second duration it permits the CA of an impaired patient to be restored within 5-30 second of the modification of the blood flow to the patient. It also permits the patient's impaired CA to be restored intact within 5-30 seconds of the determination that the patient's CA is impaired.

CA status in the current clinical trial was identified according to the shape of CA transient responses. Transient responses are the intracranial blood flow autoregulatory reactions following each front of the rectangular ABP(t) pulses as a series of CA system's input step functions.

**FIG. 1 (a)** shows rectangular ABP(t) input signals of hyperaemic **10** and hypoaemic **20** tests of CA system in the case of intact CA, while **FIG. 1 (b)** shows the CA transient responses to both the hyperaemic test **30** and hypoaemic test **40**. In response to both the hyperaemic test **30** and hypoaemic test **40** the patient's CA returns to a steady state value.

**FIG. 2 (a)** shows different rectangular ABP(t) input signals of hyperaemic **50** and hypoaemic **60** tests in the case of an impaired CA. **FIG. 2 (b)** shows no CA transient responses in the case of impaired CA. In response to the hyperaemic test **50,** the CA transient response **70** mirrors the increase in ABP. Similarly, in response to the hypoaemic test **60** the CA transient response **80** mirrors the decrease in ABP. The impaired CA has no effect on the ABP resulting in essentially no change to the shape of the CA transient responses from the shape of the ABP(t) input signals.

The patients were grouped into those who experienced POCD and those without POCD and into those who experienced post-operative delirium, and those without delirium.

The Mann-Whitney U test was applied to identify associations between patients' outcomes and the duration of longest CA impairment (LCAI) events estimated using critical Adjusted Rand Index ("ARI") =3 value by testing the statistical significance of differences between factors among groups. Fisher's exact test was used to test statistical significance for binary data (gender) among outcome groups. Other demographic data (age, sex) were also included in the analysis. Statistically significant differences were assumed when the Mann-Whitney U test results were p < 0.05. Critical threshold values of analyzed factors that significantly separated outcome groups were identified according to the chi-square maximum value. The point estimate and data distribution among groups were expressed using either the mean and standard deviation (std) or the median and interquartile range (IQR).

Tables 1 and 2 list the collected clinical and demographic data among patients' outcome groups (No-deterioration vs. POCD and No-delirium vs. Delirium). Statistically significant differences were found in age, and duration of the LCAI in patients with and without POCD, as well as in averaged mean ABP and duration of the LCAI in patients with and without delirium.

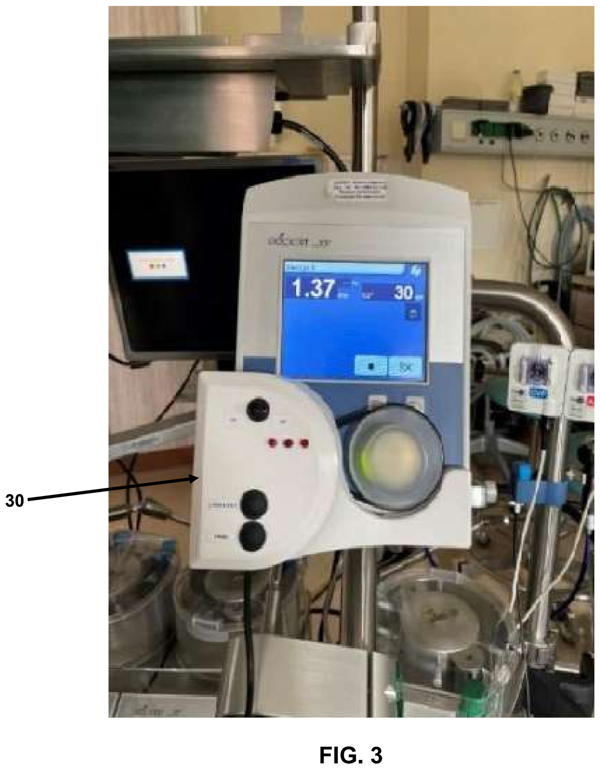

**FIG. 3** shows one embodiment of the novel mechatronic plug-in modulator device **30** for continuous modulation of blood flow with rectangular pulses having a 30-second duration device. The modulator device **30** was used during the trial for implementing the inventive mode of a heart lung machine operation-rectangular modulation of blood flow for real-time CA status monitoring and blood flow control during the CPB.

The modulator device **30** attached to the front panel of the heart and lung machine blood flow control unit regulates blood flow to generate rectangular modulation of blood flow with adjustable mean blood flow value. Such blood flow is transformed into rectangular arterial blood pressure pulsation in the patient's body with adjustable mean ABP value (mABP).

Blood flow was modulated by ± 5 % of the actual flow rate to get the amplitude of APB(t) rectangular waves of up to ± 5 mmHg. A Draeger Infinity DeltaXL monitor was used to monitor invasive ABP(t) data. Ultrasonic transcranial robotic two-channel Doppler monitor (Viasonix Dolphin 4D) was used to monitor cerebral blood flow velocity as a function of time (CBFV(t)) in the left and right middle cerebral arteries. ABP(t) and CBFV(t) data were registered by using the Intensive Care Brain Monitoring System software tool ICM+ (Cambridge, UK).

Tiecks model was used to quantify CA status based on the different shapes of transient CA responses in the CBFV(t) signal when CA is intact or impaired, quantifying such shapes by ARI values. The transient response of an intact CA system reaches a steady level within 5 to 15 seconds. This is evidence that it is possible to identify CA status periodically if the blood flow velocity of the heart and lung machine has a rectangular periodical step function shape and the period of such continuous blood flow modulation process has a pulse repetition period of 15 seconds or longer.

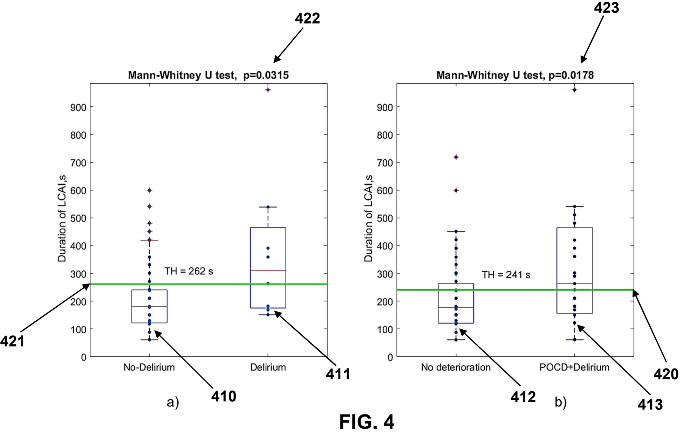

**FIG. 4** shows the CA status monitoring and identification of CA impairment events was successfully detected in patients using the inventive approach of rectangular blood flow modulation in HLM during CPB. Associations are shown in **FIG. 4** between the duration of the longest CA impairment event and postoperative delirium and POCD, including delirium. **FIGs. 4a** and **4b** shows the identification of CA impairment events by the longest duration of CA impairment (LCAI) in seconds **410, 411, 412** and **413.** The relationship between the duration of the longest CA impairment (LCAI) event and postoperative delirium is shown in **FIG. 4a** while the relationship between the duration of the LCAI event and POCD plus postoperative delirium is shown in **FIG. 4b****.** Where TH **420, 421** is a chi-square threshold indicating the best split point between two groups represented in **FIG. 4** by a box plot method, the duration of CA impairment events longer than 262 seconds **421** was associated with delirium **411** and the duration of CA impairment events longer than 241 seconds **420** as p=0.0315 **422** was associated with postoperative cognitive dysfunction plus delirium (POCDE+Delirium) as p=0.0178 **423.** Patients were grouped into those who experienced post-operative delirium **411** vs. those without delirium **410,** and those who experienced POCD including delirium **413** vs. those without signs of cognitive deterioration **412.** Thus, the duration of single CA impairment events longer than 262 seconds and 241 seconds are associated with delirium **FIG. 4 (a)** and POCD plus delerium **FIG. 4(b)** respectively.

Our analysis confirmed the associations between the longest duration of CA impairment (LCAI) with POCD and delirium. The impact of the duration of single LCAI events is also known in other studies of traumatic brain injury patients, demonstrating that patients experiencing longer CA impairment events are likely at risk of unfavorable outcomes. Statistically significant differences were found in age and duration of the LCAI in patients with and without POCD, as well as in averaged mean ABP and duration of the LCAI in patients with and without delirium. The associations between the duration of the longest CA impairment event and postoperative delirium and POCD are presented in **Fig. 4****.** The duration of CA impairment events longer than 241 sec and 262 sec were associated with POCD and delirium.

Mean arterial blood pressure (MAP) management during CPB is proposed for patient-specific continuous optimal cerebral perfusion control. Many cardiac centers maintain ABP within 50-60 mmHg according to guidelines based on evidence-based medicine concepts. This target is based on the identification of 50 mmHg as the universal evidence-based lower limit of autoregulation (LLCA), below which CA is impaired.

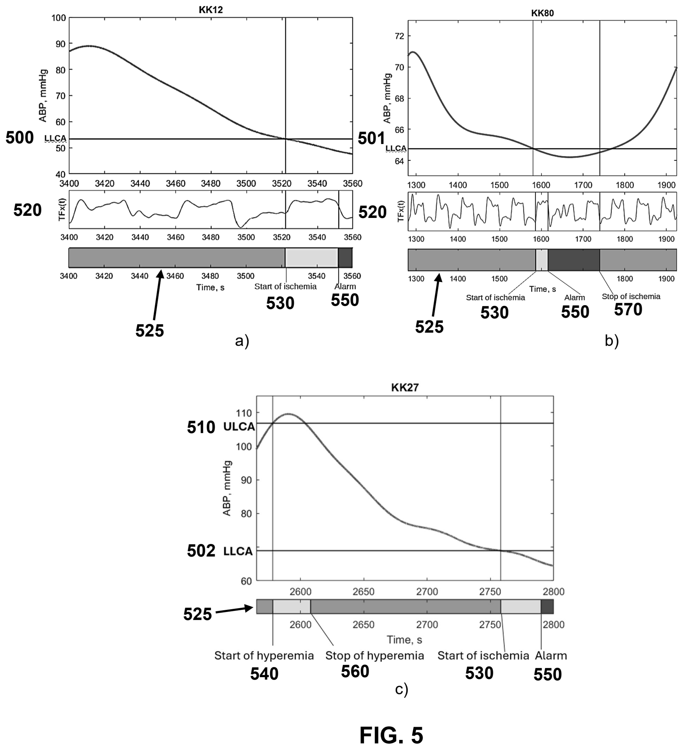

**FIG. 5** are graphs showing the lower limits of cerebral autoregulation for three different patients with impaired CA. **FIG. 5** shows real-time identification of lower limits of cerebral autoregulation (LLCA) and upper of cerebral autoregulation (ULCA), which represents the ABP values at which CA status starts to be impaired. In **FIG 5****.** TFx(t) **520** is the transient responses of CA system; colors of alarm bar - dark gray shows intact CA **525,** light gray shows ischemic (ABP<LLCA) events **530** or hyperemic (ABP>ULCA) events **540,** black - alarm signal **550** which requires intervention to restore intact CA by management of ABP. It is known that the lower ("LLCA") and upper limits of cerebral autoregulation ("ULCA") are patient-specific and vary widely. **FIG. 5** shows that LLCA **500, 501, 502** can differ for individual patients from the values recommended by evidence-based guidelines. Identified values for different patients in these examples range from approximately 53 mmHg to 70 mmHg **500, 502.** **FIGs. 5b** and **5c** also show the cases when CA status is recovered by ABP mean value management **560, 570.**

Maintaining mean arterial blood pressure within the ranges recommended by guidelines might be sub-optimal for specific individuals, potentially leading to perioperative cerebral injury. For elderly patients or those at high risk (e.g., those with hypertension, pre-existing cerebrovascular disease, or diabetes), some studies recommend maintaining mean arterial blood pressure above 70 mmHg. Conversely, a higher mean arterial blood pressure could exceed an individual's upper limit for CA, resulting in cerebral hyperemia, which has been linked to postoperative delirium. Use of the invention support this, as elevated ABP is associated with an increased risk of postoperative delirium (p = 0.0017, Table 1).

Many studies have suggested that ABP targets in general anesthesia should be personalized and this has been confirmed by the invention. By implementing rectangular blood flow modulation and blood flow control in the HLM machine the invention demonstrates the real-time identification of CA impairment events, which allows the management of mean blood flow with a minimal delay (in seconds) as well as to restore brain perfusion promptly after the alarm signal of CA impairment is generated **(****Figs. 5b,5c****).** The restoration of optimal brain perfusion physiologically means a restoration of an individual patient-specific optimal perfusion in all his vital organs and especially to the brain.

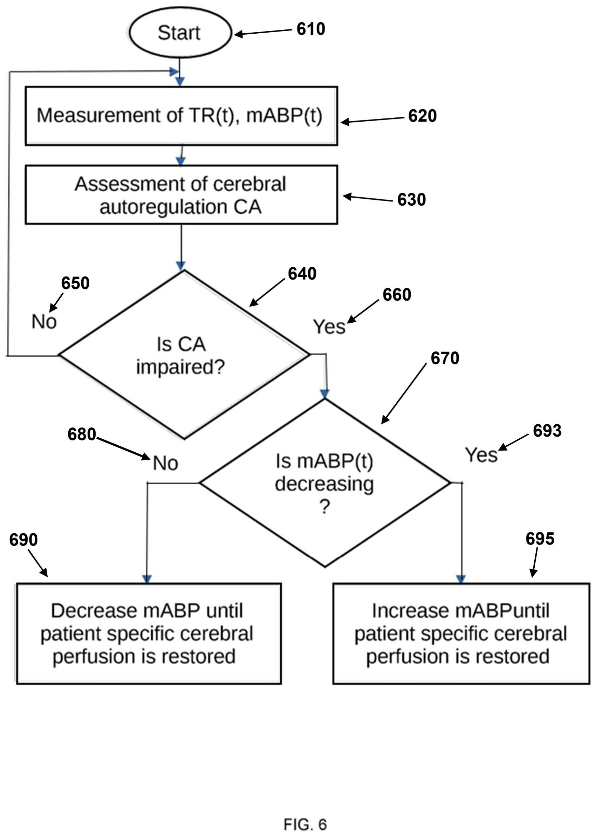

**FIG. 6** is an algorithm of the method for using mean ABP (mABP) management in order to restore patient specific cerebral perfusion in a matter of seconds. Preferably, the patient's specific cerebral profusion is restored in less than 5-10 seconds. As shown in **FIG. 6****,** the method starts **610** with connecting a patient to a heart lung machine and delivering blood to the patient with a periodic rectangular blood flow modulation having a pulse repletion period of between 15 to 30 seconds. The next step is measuring of the patients TR(t) and mean arterial blood pressure over time (mABP(t) **620.** The is followed by an assessment of the patient's cerebral autoregulation, (CA) state. **630.** The assessment is whether or not the patient CA is impaired **640.** If the patient CA is determined to be not impaired **650** then the method returns to continuing to measure the patient's TR(t) and mABP(t) **620.**

If it is determined that the patient CA is impaired **660,** then the next step of the method is to determine if the patient's mABP is decreasing over time **670.** If the mABP is not decreasing over time **680** then the heart lung machine decreases the pressure until the mABP of the patient's specific cerebral perfusion is restored **690.** Alternatively, if the mABP of the patient is decreasing over time **693,** then the heart lung machine increases the pressure until the patient specific cerebral perfusion is restored **693.**

The invention demonstrates that an individual patient-specific lower and upper limits of cerebral blood flow autoregulation can be identified in real-time. It also has been shown that the start and stop moments of brain injury caused by excursions of ABP outside the patient-specific limits of cerebral autoregulation can also be identified in almost real-time with a potentially minimal 15-second delay of the alarm signal. Precise personal ABP(t) management can be performed during CPB to restore patient-specific optimal brain perfusion with sub-minute time resolution and reduce the incidence of postoperative cognitive dysfunction and delirium.

One embodiment of the system comprises a heart-lung machine configured to deliver blood to the patient with periodic rectangular modulation having a pulse repetition period of 15 to 30 seconds. It also includes an arterial blood pressure monitor configured to obtain ABP(t) signals comprising discrete rising and falling fronts downstream of the patient. An intracranial blood flow velocity monitor is also included in the system and it is configured to obtain cerebral autoregulation transient responses following each discrete front of the ABP(t) signals downstream of the patient. The embodiment further includes a processor configured to evaluate each transient response to derive an autoregulatory index and compare the index to a predetermined impairment threshold to detect cerebral autoregulation impairment events, and to identify patient-specific lower and upper arterial blood pressure limits at which cerebral autoregulation impairment occurs based on the detected impairment events. The processor is further configured, upon detecting an impairment event, to modify the periodic rectangular blood flow modulation of the heart-lung machine to adjust the mean arterial blood pressure of the patient to within the identified patient-specific limits.

It should be appreciated that the various systems and methods described above are embodiments and are provided by way of illustration only and should not be construed as limiting the invention. Numerous changes and modifications may be made from the embodiments described above without departing from the spirit and scope of the invention. As such, it is intended that the invention not be limited by the particular embodiments disclosed. Various features of the present invention are set forth in the following claims.

## Claims

1. A system for use during coronary artery bypass grafting surgery comprising a heart lung machine, and a modulator (30) connected to the heart lung machine configured to modulate the blood flow from the heart lung machine to a patient with a periodic rectangular blood flow modulation;
a first monitor configured for monitoring the modulation of the periodic rectangular blood flow and measuring rectangular ABP(t) input signals (10, 20, 50, 60), the ABP(t) input signals (10, 20, 50, 60) having a front (1, 2, 3, 4, 5, 6, 7, 8);
a second monitor configured for monitoring intracranial blood flow or volume auto regulatory reactions cerebral autoregulation (CA) transient responses (70, 80) following the front (1, 2, 3, 4, 5, 6, 7, 8) of the rectangular ABP(t) input signals (10, 20, 50, 60); and
a processor configured to compare the rectangular blood flow modulation to the ABP(t) and to determine if the cerebral autoregulation (CA) of the patient is impaired based on the comparison.

2. The system of claim 1 wherein the periodic rectangular blood flow has a period of between 15 and 30 seconds.

3. The system of claim 1 wherein the modulator (30) is configured to alter the modulation of blood flow from the heart lung machine when the CA of the patient is impaired.

4. The system of claim 3, wherein the modulator (30) is configured to modulate the blood flow by ±5% of the actual flow rate to generate rectangular ABP(t) waves with an amplitude of up to ±5 mmHg.

5. The system of claim 1, wherein the modulator (30) is configured to provide an adjustable mean blood flow value during the periodic rectangular blood-flow modulation.

6. The system of claim 1, wherein the processor is further configured to generate an alert (550) when it is determined the CA impairment (530, 540) of the patient is detected having a duration exceeding 241 seconds.

7. The system of claim 1, wherein the processor is further configured to generate an alert (550) when it is determined the CA impairment (530, 540) of the patient is detected having a duration exceeding 262 seconds.

8. A system for real-time determination and restoration of cerebral autoregulation in a patient undergoing cardiopulmonary bypass, comprising:
a heart-lung machine configured to deliver blood to the patient with periodic rectangular modulation having a pulse repetition period of 15 to 30 seconds;
an arterial blood pressure monitor configured to obtain ABP(t) signals (10, 20, 50, 60) comprising discrete rising (1, 3, 5, 7) and falling fronts (2, 4, 6, 8) downstream of the patient;
an intracranial blood flow velocity monitor configured to obtain cerebral autoregulation transient responses (70, 80) following each discrete front (1, 2, 3, 4, 5, 6, 7, 8) of the ABP(t) signals (10, 20, 50, 60) downstream of the patient;
a processor configured to evaluate each transient response (70, 80) to derive an autoregulatory index and compare the index to a predetermined impairment threshold to detect cerebral autoregulation impairment events (530, 540), and to identify patient-specific lower and upper arterial blood pressure limits (500, 501, 502, 510) at which cerebral autoregulation impairment occurs based on the detected impairment events (530, 540);
wherein the processor is further configured, upon detecting an impairment event (530, 540), to modify the periodic rectangular blood flow modulation of the heart-lung machine to adjust the mean arterial blood pressure of the patient to within the identified patient-specific limits (500, 501, 502, 510).

9. A system for use during coronary artery bypass grafting surgery, comprising:
a heart-lung machine;
a modulator (30) connected to the heart-lung machine configured to modulate the blood flow from the heart-lung machine to a patient with a periodic rectangular blood-flow modulation;
a first monitor configured for monitoring the periodic rectangular blood-flow modulation and measuring rectangular ABP(t) input signals (10, 20, 50, 60), the ABP(t) input signals (10, 20, 50, 60) having a front (1, 2, 3, 4, 5, 6, 7, 8);
a second monitor configured for monitoring intracranial blood-flow or volume autoregulatory reactions CA transient responses (70, 80) following the front (1, 2, 3, 4, 5, 6, 7, 8) of the rectangular ABP(t) input signals (10, 20, 50, 60); and
a processor configured to compare the rectangular blood-flow modulation to the ABP(t) (10, 20, 50, 60) and to determine if the CA of the patient is impaired based on the comparison.

10. The system of claim 9, wherein the period of the periodic rectangular blood flow is between 15 and 30 seconds.

11. The system of claim 9, wherein the modulator (30) is configured to alter the modulation of the blood flow from the heart-lung machine when the CA of the patient is impaired.

12. The system of claim 9, wherein the processor is further configured to generate an alert (550) when it is determined the CA impairment (530, 540) of the patient is detected having a duration exceeding 241 seconds.

13. The system of claim 9, wherein the processor is further configured to generate an alert (550) when it is determined the CA impairment (530, 540) of the patient is detected having a duration exceeding 262 seconds.

14. A method for determining CA impairment of a patient during coronary artery bypass grafting surgery comprising:
attaching (610) a heart lung machine with a modulator (30) to the patient;
transmitting blood to the patient using periodic rectangular blood flow modulation;
monitoring (620) the rectangular periodic blood flow modulation and measuring rectangular ABP(t) input signals (10, 20, 50, 60) of the patient's cerebral autoregulation system;
monitoring (630) the intracranial blood flow auto regulatory reactions CA transient responses (70, 80) of the patient following the front (1, 2, 3, 4, 5, 6, 7, 8) of the rectangular ABP(t) input signals (10, 20, 50, 60); and
comparing (640) the rectangular blood flow modulation to the ABP(t) (10, 20, 50, 60) with the CA transient responses (70, 80) of the patient to determine if the patient's CA is impaired.

15. The method of claim 14 further including the step of modifying the rectangular blood flow from the heart lung machine when the CA of the patient is impaired (660) to restore intact CA and a patient specific brain perfusion in seconds by management (670) of mean ABP(t).

16. The method of claim 15 wherein the CA of the patient is restored within 5-30 seconds of the modification of the blood flow from the heart lung machine.

17. The method of claim 15 wherein the impaired CA of the patient is restored within 5-30 seconds of determining the patient's CA is impaired.

18. The method of claim 14, wherein the periodic rectangular blood-flow modulation is performed by modulating the blood flow by ±5% of the actual flow rate to generate rectangular ABP(t) waves with an amplitude of up to ±5 mmHg.

19. The method of claim 14, wherein the periodic rectangular blood-flow modulation includes adjusting (690, 695) the mean blood flow valueduring the modulation.

20. A method for real-time determination and restoration of cerebral autoregulation in a patient undergoing cardiopulmonary bypass, comprising:
operating (610) a heart-lung machine to deliver blood to the patient with periodic rectangular modulation having a pulse repetition period of 15 to 30 seconds;
monitoring (620) invasive arterial blood pressure downstream of the patient to obtain ABP(t) signals (10, 20, 50, 60) comprising discrete rising (1, 3, 5, 7) and falling (2, 4, 6, 8) fronts;
monitoring (630) intracranial blood flow velocity downstream of the patient to obtain cerebral autoregulation transient responses (70, 80) following each discrete front (1, 2, 3, 4, 5, 6, 7, 8) of the ABP(t) signals (10, 20, 50, 60);
evaluating each transient response (70, 80) to derive an autoregulatory index and comparing said index to a predetermined impairment threshold to detect (660) cerebral autoregulation impairment events (530, 540);
identifying patient-specific lower and upper arterial blood pressure limits (500, 501, 502, 510) at which cerebral autoregulation impairment occurs based on the detected impairment events (530, 540); and
upon detecting an impairment event (530, 540), modifying the periodic rectangular blood flow modulation of the heart-lung machine to adjust (690, 695) the mean arterial blood pressure of the patient to within the identified patient-specific limits (500, 501, 502, 510).

21. The method of claim 20, further comprising generating an alert (550) if a detected cerebral autoregulation impairment event (530, 540) persists for more than 241 seconds.

22. The method of claim 20, further comprising generating an alert (550) if a detected cerebral autoregulation impairment event (530, 540) persists for more than 262 seconds.

23. The method of claim 20, wherein the CA of the patient is restored within 5-30 seconds of the modification (690, 695) of the blood flow from the heart-lung machine.

24. The method of claim 20, wherein the modification of the periodic rectangular blood-flow modulation comprises automatically adjusting at least one of amplitude, duty cycle, or baseline of the rectangular waveform so that the patient's mean arterial pressure is shifted into the patient-specific range previously identified as compatible with intact autoregulation, thereby reestablishing cerebral autoregulation within no more than 30 seconds from detection.
